# EUROPEAN PATENT SPECIFICATION

(11) **EP 2 818 193 B1**
(45) Date of publication and mention of the grant of the patent: **18.04.2018**
(21) Application number: 13173922.9
(22) Date of filing: 27.06.2013
(51) Int. Cl.: A61M 16/06

(54) **Respiratory mask with detachable holding arm**
Atemmaske mit abnehmbarem Haltearm
Masque respiratoire avec bras de maintien amovible

(43) Date of publication of application: 31.12.2014
(73) Proprietor: Air Liquide Medical Systems, 92160 Antony (FR)
(72) Inventor: Alberici, Luca, 25128 BRESCIA (IT); Masserdotti, Fulvio, 25075 BRESCIA (IT); Sandoni, Giuseppe, 25124 BRESCIA (IT)
(74) Representative: Pittis, Olivier

(56) References cited:
- EP-A1- 1 225 945
- WO-A1-98/04310
- WO-A1-99/21618
- WO-A1-2005/016142
- WO-A1-2012/028994
- FR-A1- 2 735 030
- US-A1- 2003 145 857
- US-A1- 2005 051 171
- US-A1- 2009 320 850

## Description

The invention concerns a respiratory mask, in particular a nasal mask, in particular for adult or pediatric uses in the treatment of respiratory conditions or diseases affecting adults, children, toddlers, infants or newborns, comprising a holding arm with a detachable distal portion.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD), etc.

Nasal masks deliver a flow of breathable gas for, or to assist in, patient respiration.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face, which cushion is usually made of soft, resilient elastomeric material, such as soft silicone or similar, and a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

However, with the current masks, it is often difficult to obtain an efficient positioning of the mask on the patient's face and to ensure sufficient tightness (seal) preventing the escape of gas as well as a comfort of use for the patient.

For instance, some masks including various mechanisms for positioning the forehead support are disclosed by US-A-2245658, EP-A-1985327 and EP-A-1356842.

US-A-2009/320850 discloses a respiratory mask according to the preamble of claim 1, including a holding arm projecting upwardly from the mask body, and comprising a free end with means for fixing a headgear thereto.

Alternative respiratory mask structures are disclosed by WO-A-98/04310, WO-A-2005/016142 and WO-A-99/21618.

Furthermore, EP-A-1225945 and US-A-2003/145857 teach respiratory masks comprising a mask body with a holding arm and two lateral arms that are molded in one piece with the mask body.

However, so far, while attempts for modifying the existing positioning mechanisms have been made, the resulting masks are not totally satifactory.

Indeed, as the morphology of the face and the need is different from one user to another, it is important to be able to correctly position the forehead support of the mask on the user's forehead and afterwards maintain it in a desired position, whatever the forehead morphology of the patient is.

This is true with adult patients but also particularly true with toddlers, infants, newborns and children. Thus, the facial morphologies of children or toddlers of one or several years and of infants/babies of several days, weeks or months are very dissimilar, i.e. very different, as they change very quickly while the infant/child is growing up.

This becomes an important problem when the patient should receive a gaseous treatment delivered by means of a respiratory mask as the mask to be used should well-fit with any facial morphology in order to get an efficient gas delivery.

For instance, the holding arm carrying the forehead support of current masks can be perfectly suitable for some patients but, in contrast, a real problem with other patients, such as infants/small children, because these masks cannot be not well-adapted to some facial morphologies due to their size.

Indeed, the size of the holding arm can be too big and very cumbersome compared to the size of the head of some patients.

This could lead to some difficulties for well-positioning the mask on the face of said patients and maintaining it thereafter without gas leaks, as the forehead support does not contact and rest at all, or only imperfectly, on the forehead of the patient due to its excessive size.

In other words, with current masks, it is often difficult to obtain an efficient positioning and securing of the mask on the patient's face and to ensure a sufficient tightness (seal) preventing the escape of gas as well as a good comfort of use for the patient, especially when the patient is an infant, such as a newborn or a baby, a toddler or a child.

Hence, the problem to be solved is to provide an improved mask architecture, especially a nasal mask, allowing an easy and efficient positioning of the mask body on the patient's face so as to ensure efficient gas tightness (seal) and/or increase comfort for the user, whatever the user, including infants, children or similar having variable facial morphologies. The invention is defined by the appended claim 1. The solution of the present invention concerns a respiratory mask, in particular a nasal mask, preferably a pediatric mask, comprising a mask body and a holding arm arranged on the mask body and projecting upwardly from said mask body, said holding arm comprising a free end, the holding arm further comprising at least a proximal portion and a distal portion, said proximal portion being integrally fixed to the mask body and said distal portion comprising the free end of said holding arm, the distal portion being detachable from the holding arm, the proximal portion and the distal portion of the holding arm each comprising fixing means for fixing a headgear, characterized in that:
- the mask body and the holding arm are molded in one piece and made of a resilient soft material, and
- the intermediary area between the proximal portion and the distal portion comprises a neck portion forming a neck having a first thickness Tn less than a second thickness Tp of the proximal portion and less than a third thickness Td of the distal portion.

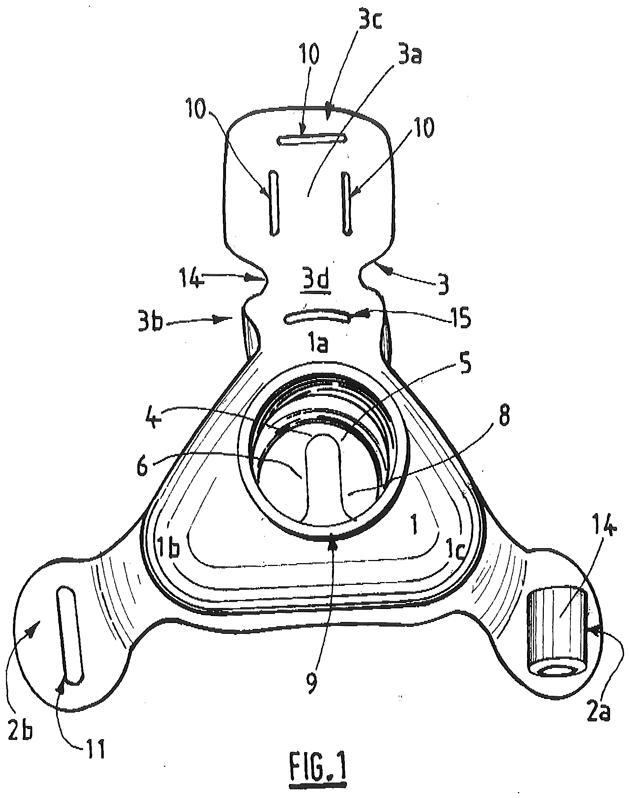

The mask according to the present disclosure can further comprise one or more of the following additional features:
- the distal portion of the holding arm is conformed or adapted for being easily detachable from the holding arm.
- the detachment of the distal portion from the holding arm is achieved by cutting or breaking at least a part of said holding arm comprising said distal portion.
- the proximal portion and the distal portion are separated by an intermediary area comprising the location of detachment of the distal portion.
- the neck portion comprises the location (region, zone, site...) of detachment of the distal portion.
- the holding arm is made (at least in part) of a resilient soft or semi-soft material.
- the mask body and the holding arm are molded in one piece and made of a resilient soft or semi-soft material.
- the resilient soft material comprises silicone or similar.
- the proximal portion and the distal portion of the holding arm each comprise fixing means for fixing a headgear that is used for maintaining and securing the mask in a desired position on the head of the patient.
- the fixing means are traversing holes or slots, preferably slots. For instance, the slots can have a width of between 8 to 25 mm.
- the mask body further comprises an internal chamber and an inlet orifice in fluid communication with the internal chamber.
- the mask body further comprises a flexible cushion having an aperture for receiving at least part of the patient's nose, when the patient wears the mask.
- the mask body further comprises two lateral arms arranged on each side of the mask body and projecting laterally from said mask body. One of the two lateral arms projects on the right side of the mask body, whereas the other one projects on the left side of the mask body, when considering a front view of the mask (as illustrated on Fig. 1).
- the mask body, the holding arm, the cushion and the two lateral arms are integral and form a unique piece made of a resilient soft or semi-soft material.
- the cushion comprises at least one flexible membrane.
- the membrane and the cushion are integral and formed of a unique piece made of a resilient soft or semi-soft material, i.e. of silicone or similar.
- the two opposite lateral arms projecting laterally from said mask body comprises headgear connection means for fixing a headgear.
- the headgear connection means comprise at least a (i.e. one or more) traversing hole or slot.
- the mask further comprises a headgear fixed to the headgear connection means and to the fixing means.
- the headgear is fixed to the mask by means of one or several straps. The straps are made of fabric or polymer.
- the mask body has a general triangular tridimensional shape.
- the inlet orifice is arranged at the center of the mask body.
- the two opposite lateral arms have a generally curved-shape.
- the mask is a nasal mask, preferably it is pediatric nasal mask dimensioned for fitting to the nasal region of an infant or a child.

The invention also concerns an assembly comprising a gas delivery device, such as a medical ventilator, and a respiratory mask according to the present invention, preferably the gas delivery device is connected to the respiratory mask by means of a gas line, such as a flexible hose.

A preferred embodiment of a nasal mask according to the present invention is shown in the enclosed Figures, among which:
- Figure 1 represents a front view of an embodiment of a respiratory mask according to the present invention,
- Figure 2 is a lateral view of the mask of Figure 1,
- Figure 3 is a rear view of the mask of Figure 1,
- Figure 4 shows the tubing elements fixed to the mask of Figure 1 and
- Figure 5 is similar to Figure 1 but shows the distal portion of the holding arm detached from the mask body.

The facial morphologies of some adults and especially of children/toddlers of one or several years and of infants/babies of several days, weeks or months are very dissimilar, i.e. very different, as they change very quickly while the infant/child is growing up. This becomes an important problem when the child/infant should receive a gaseous treatment delivered by means of a respiratory mask because the mask to be used should well-fit with any facial morphology in order to get an efficient gas delivery.

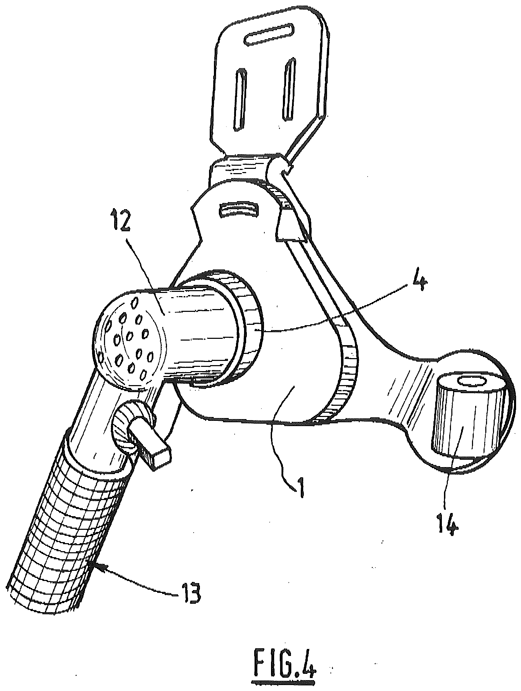

In order to overcome those problems, the present invention proposes a new type of nasal mask as illustrated in Figures 1 to 5 that illustrate one embodiment of a pediatric nasal mask according to the present invention.

Generally speaking, a respiratory mask according to the present disclosure comprises a hollow shell or mask body 1 defining an internal breathing chamber 6 or volume, wherein respiratory gas, such as air under pressure, is introduced via an inlet port 4 to which is connected a gas feeding line 13, such as a flexible hose, by means of a tubular connector 12 or similar.

The gas inlet orifice 4 is arranged at the center of front side of the mask body 1 and through its wall thereby allowing gas under pressure to be introduced in the breathing chamber 6, said inlet orifice 4 being in fluid communication with the internal chamber 6.

In other words, the shell or mask body 1 is fluidly linked to a gas supply line 13, such as a flexible hose, by means of the tubular hollow connector 12, which delivers a respiratory gas, such as air under pressure, into the breathing chamber 6 of the mask body 1. The gas supply line is fed with gas under pressure by a respiratory device or ventilator (not shown).

The tubular hollow connector 12 is fixed to the mask body 1 by means of an annular element 9, such a polymer (e.g. plastic) ring or similar structure, that is arranged in the inlet orifice 4 of the mask body 1 that is located in the front side of the mask body.

The mask body 1 has preferably a generally triangular tridimensional shape, as visible in Figure 1 for example, so as to better match the nasal regions of the patient. However, the mask body 1 can have another tridimensional shape.

The mask body 1 is configured to a pediatric usage. It receives at least a part of the patient's nose, when said patient introduces his/her nose into the internal volume of the breathing chamber 6 of the mask body 1 and breathes the gas contained therein.

Further, the mask body also comprises, on the one hand, a holding arm 3 forming an upper or frontal support that is integral with the mask body 1 and projects upwardly from said mask body 1 and two lateral arms or wings 2a, 2b that are also integrally arranged on the mask body 1 and that project laterally, i.e. on each opposite lateral sides (right and left sides) from said mask body 1 so as to constitute right and left cheek supports, when the mask is worn by a patient, as illustrated in Figures 1-3 and 5.

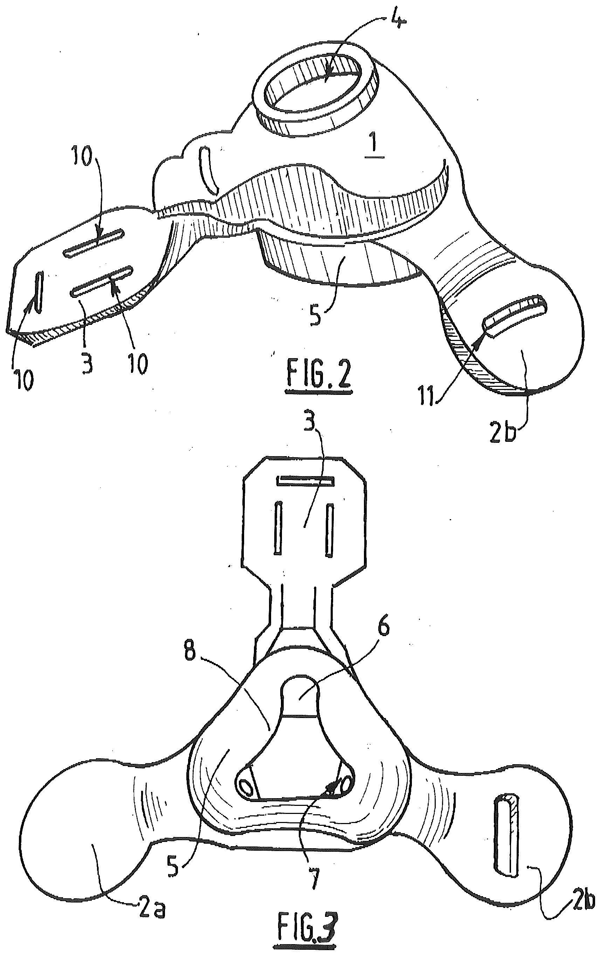

Furthermore, a cushion 5 having an aperture 7 for receiving at least part of the patient's nose, when the patient wears the mask, is arranged on the rear side of the mask body 1 as shown in Figure 3.

Preferably, in order to provide efficient gas tightness (seal) and/or increased comfort for the patient, when said patient is a infant or child, the mask body 1, the holding arm 3, the cushion 5 and the two lateral arms 2a, 2b are made of a single piece made of a resilient soft or semi-soft material, such as preferably silicone. Preferably, they are molded in one piece.

Said single piece of resilient soft or semi-soft material being entirely flexible, it can be adapted to a great variety of facial morphologies, especially of various nasal regions of patients, in particular infant or child patients.

During use of the mask, the soft or semi-soft resilient cushion 5 comes into contact with the patient's face. The central aperture 7 receiving at least a part of the patient's nose, can have a triangular or similar shape or structure as illustrated in Figure 3, so as to match the contours of the nasal region of the patient.

A soft flexible membrane 8 forming a kind of skirt delimiting said central aperture 7, is molded in one piece with the rest of the cushion 5 so as to form part of the single piece of flexible material.

In that case, the cushion 5 and the membrane 8 comprise an upper nasal bridge region, a lower region and two lateral regions (i.e. left and right regions) connecting the nasal bridge and lower regions. When the mask is worn by the patient, the upper nasal bridge region is in contact with the nasal bridge of the patient, the lower region is in contact with the area between the nose and the upper lip of the patient, and the lateral opposite regions are in contact with the flange regions of the nose of the patient.

Alternatively, the cushion 5 can also comprise several membranes 8, such as two superimposed membranes. Using several membranes may improve the gas tightness. Nevertheless, in the present embodiment, a single membrane is provided.

Further, in the embodiment of Figure 1, the mask body 1 according to the present invention has a generally triangular tridimensional shape that more or less matches or corresponds to the general structure of a nose. Said generally triangular tridimensional shape or structure comprises three angle areas 1a-1c forming the three corners of the triangular structure of the mask body 1.

The holding arm 3 projects upwardly from one 1a of the three angle areas 1a-1c of said mask body 1, whereas the two lateral arms 2a, 2b project laterally from the two other angle areas 1b, 1c of said mask body 1 as illustrated in Figure 1.

The holding arm 3 comprises at least a proximal portion 3b and a distal portion 3a. The proximal portion 3b is the part of the holding arm that is integrally fixed to the mask body 1, whereas the distal portion 3a is situated at the opposite end of the hold arm 3 and carries the free end 3c of said holding arm 3.

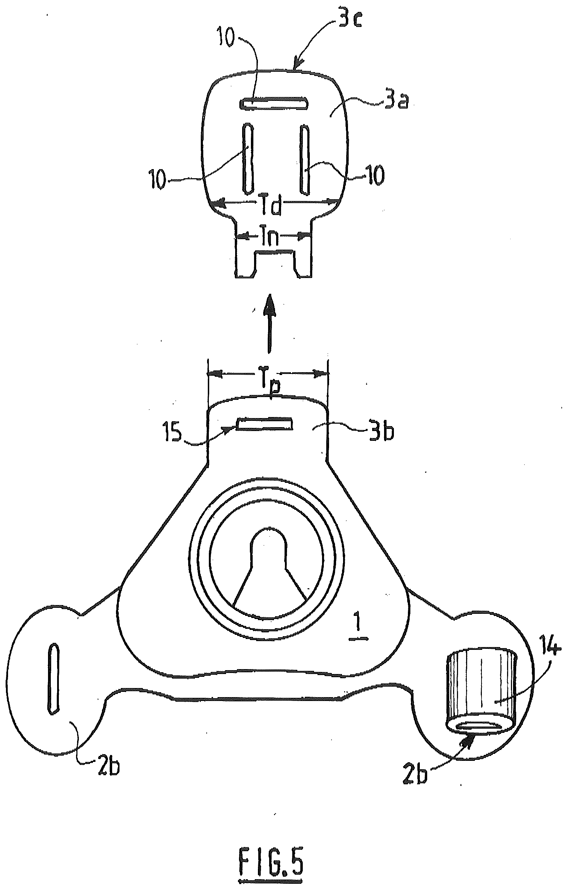

According to the present invention, the distal portion 3a of the holding arm 3 is detachable from the rest of the holding arm 3. In other words, the distal portion 3a of the holding arm 3 can be removed from the holding arm 3, if the physician or other medical provider or the patient desires to do so. Such a configuration is illustrated in Figure 5, where one can see that the distal portion 3a of the holding arm 3 has been removed by cutting.

The detachment of the distal portion 3a from the holding arm 3 can be done in different ways, but preferably it is operated by cutting or by breaking the part of the holding arm 3 that comprises said distal portion 3a. Most preferably, the detachment of the distal portion 3a is done by cutting it, for instance by means of scissors, a knife, a scalpel or any another cutting tool.

It can be desired to remove the distal portion 3a of the holding arm 3 in various situations and/or for different reasons. For instance, it can be removed when the mask is used with little children due to their facial morphology, but also with adults that feel better when wearing a lighter mask, especially during the day. Indeed, the distal portion 3a of the holding arm 3 of the mask adds some weight and volume to the mask, which can be troublesome for some patients, in particular when they read a book or watch a screen or a monitor, such as computer, TV.... Further, some physicians prefer to remove the distal portion 3a of the holding arm 3 because they believe that, without the distal portion 3a of the holding arm 3, the gas therapy is better accepted and followed by their patients.

Preferably, the detachment region is or comprises an intermediary area 3d located between the proximal portion 3b and the distal portion 3a. Said intermediary area 3d comprises a neck portion 14 forming a neck having a first thickness Tn less than the second thickness Tp of the proximal portion 3b and less than the third thickness Td of the distal portion 3a, as illustrated in Figure 5, thereby facilitating the detachment and removal, i.e. by cutting, of the part of the holding arm 3 comprising the distal portion 3a. The second thickness Tp and the third thickness Td can be equal or different.

As a non-limitative example, the neck can have a first thickness Tn of between 10 and 20 mm, whereas the proximal portion 3b can have a second thickness Tp of between 15 and 25 mm and the distal portion 3a can have a third thickness Td of between 24 and 34 mm, with Tn < Tp and Tn < Td.

Indeed, if a neck portion 14 forming a neck, or similar narrowed structure, is arranged on the holding arm 3, it is easier to cut the latter in the area comprising that neck, for thereafter detaching and removing the distal portion 3a.

Preferably, the entire holding arm 3, including the proximal portion 3b, the intermediary area 3d and the distal portion 3a, is made in one piece, such as by molding of a soft flexible material, such as a silicone material.

In another example, the intermediary area 3d is a groove or similarly thinner structure -made in a different material having a lower strength than the proximal portion 3b and the distal portion 3a so as to be more easily breakable by a physician other medical provider or the patient, thereby removing the distal portion 3a as explained above.

Further, the proximal portion 3b and the distal portion 3a of the holding arm 3 each comprise fixing means 10, 15 for fixing a headgear, such as traversing holes or slots.

Preferably, the headgear is fixed to the mask by means of one or several straps (not shown) that passes through either slots 10 or slot 15, or holes having other shapes.

When the mask is as illustrated on Figure 1, i.e. with an entire holding arm 3 comprising both distal and proximal portions 3a, 3b, the straps of the headgear pass through the slots 10 of the distal portion 3a and the headgear connection means 11, 14 carried by the two opposite lateral arms 2a, 2b, which also comprise one or several traversing holes or slots 11 for receiving straps and/or one or several hollow connecting structure 14 having for example a semi-cylindrical shape and projecting away on the front surface of a arm, and comprising an inner passage for receiving the hook of a hook connector fixed to the strap of a headgear.

However, when the distal portion 3a of the holding arm 3 has been cut off and removed from the holding arm 3, the straps of the headgear pass through the slots 15 traversing the proximal portion 3b of the mask, and are further fixed to the headgear connection means 11 as in the previous case.

The straps of the headgear can be made of fabric or polymer. They are used for maintaining the mask in a desired position on the head of the patient during its use and thus obtaining an efficient treatment of respiratory disorders.

The nasal respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask comprising a mask body (1) and a holding arm (3) arranged on the mask body (1) and projecting upwardly from said mask body (1), said holding arm (3) comprising a free end (3c), the holding arm (3) further comprising at least a proximal portion (3b) and a distal portion (3a), said proximal portion (3b) being integrally fixed to the mask body (1) and said distal portion (3a) comprising the free end (3c) of said holding arm (3), the distal portion (3a) being detachable from the holding arm (3), the proximal portion (3b) and the distal portion (3a) of the holding arm (3) each comprising fixing means (10, 15) for fixing a headgear, **characterized in that**:
- the mask body (1) and the holding arm (3) are molded in one piece and made of a resilient soft material, and
- the intermediary area (3d) between the proximal portion (3b) and the distal portion (3a) comprises a neck portion (14) forming a neck having a first thickness (Tn) less than a second thickness (Tp) of the proximal portion (3b) and less than a third thickness (Td) of the distal portion (3a).

2. Respiratory mask according to the preceding Claim, **characterized in that** the proximal portion (3b) and the distal portion (3a) are separated by an intermediary area (3d) comprising a location of detachment of the distal portion (3a).

3. Respiratory mask according to any one of the preceding Claims, **characterized in that** the neck portion (14) comprises the location of detachment of the distal portion (3a).

4. Respiratory mask according to any one of the preceding Claim, **characterized in that** the resilient soft material comprises silicone.

5. Respiratory mask according to any one of the preceding Claims, **characterized in that** the fixing means (10, 15) are traversing holes or slots.

6. Respiratory mask according to any one of the preceding Claims, **characterized in that** the mask body (1) further comprises :
- an internal chamber (6) and an inlet orifice (4) in fluid communication with the internal chamber (6),
- a flexible cushion (5) having an aperture (7) adapted for receiving at least part of the patient's nose, when the patient wears the mask, and
- two lateral arms (2a, 2b) arranged on each side of the mask body (1) and projecting laterally from said mask body (1).

7. Respiratory mask according to Claim 6, **characterized in that** the mask body (1), the holding arm (3), the cushion (5) and the two lateral arms (2a, 2b) are integral and formed of a single piece made of a resilient soft material.

8. Respiratory mask according to Claim 6, **characterized in that** the two opposite lateral arms (2a, 2b) projecting laterally from said mask body (1) each comprise headgear connection means (11) for fixing a headgear, preferably the headgear connection means (11) comprise at least one traversing hole or slot.

9. Respiratory mask according to any one of the preceding Claims, **characterized in that** the mask body (1) has a general triangular tridimensional shape.

10. Respiratory mask according to any one of the preceding Claims, **characterized in that** the respiratory mask is a nasal mask, preferably a pediatric nasal mask dimensioned for fitting to the nasal region of an infant or a child.

11. Assembly comprising a gas delivery device and a respiratory mask according to any one of the preceding Claims, preferably the gas delivery device is connected to the respiratory mask by means of a gas line, such as a flexible hose.

## Patentansprüche

1. Atemmaske, umfassend einen Maskenkörper (1) und einen am Maskenkörper (1) angeordneten und vom Maskenkörper (1) nach oben projizierenden Trägerarm (3), wobei der Trägerarm (3) ein freies Ende (3c) umfasst, wobei der Trägerarm (3) des Weiteren mindestens einen proximalen Abschnitt (3b) und einen distalen Abschnitt (3a) umfasst, wobei der proximale Abschnitt (3b) am Maskenkörper (1) integriert befestigt ist und der distale Abschnitt (3a) das freie Ende (3c) des Trägerarms (3) umfasst, wobei der distale Abschnitt (3a) vom Trägerarm (3) abnehmbar ist, wobei der proximale Abschnitt (3b) und der distale Abschnitt (3a) des Trägerarms (3) jeweils Befestigungsmittel (10, 15) zum Befestigen eines Kopfgestells umfassen, **dadurch gekennzeichnet, dass**:
- der Maskenkörper (1) und der Trägerarm (3) in einem Teil geformt und aus einem elastischen weichen Material hergestellt sind, und
- der Zwischenbereich (3d) zwischen dem proximalen Abschnitt (3b) und dem distalen Abschnitt (3a) einen Halsabschnitt (14) umfasst, der einen Hals bildet, der eine erste Dicke (Tn) umfasst, die geringer ist als eine zweite Dicke (Tp) des proximalen Abschnitts (3b) und geringer als eine dritte Dicke (Td) des distalen Abschnitts (3a).

2. Atemmaske nach dem vorausgehenden Anspruch, **dadurch gekennzeichnet, dass** der proximale Abschnitt (3b) und der distale Abschnitt (3a) durch einen Zwischenbereich (3d) getrennt sind, der eine Stelle zum Ablösen des distalen Abschnitts (3a) umfasst.

3. Atemmaske nach einem der vorausgehenden Ansprüche, **dadurch gekennzeichnet, dass** der Halsabschnitt (14) die Stelle zum Ablösen des distalen Abschnitts (3a) umfasst.

4. Atemmaske nach einem der vorausgehenden Ansprüche, **dadurch gekennzeichnet, dass** das elastische weiche Material Silikon umfasst.

5. Atemmaske nach einem der vorausgehenden Ansprüche, **dadurch gekennzeichnet, dass** die Befestigungsmittel (10, 15) durchgehende Löcher oder Schlitzen sind.

6. Atemmaske nach einem der vorausgehenden Ansprüche, **dadurch gekennzeichnet, dass** der Maskenkörper (1) des Weiteren umfasst:
- eine Innenkammer (6) und eine Einlassmündung (4) in Flüssigkeitskommunikation mit der Innenkammer (6),
- ein flexibles Kissen (5), das eine Öffnung (7) hat, die angepasst ist, um mindestens einen Teil der Nase des Patienten aufzunehmen, wenn der Patient die Maske trägt, und
- zwei an jeder Seite des Maskenkörpers (1) angeordnete und vom Maskenkörper (1) seitwärts projizierende Seitenarme (2a, 2b).

7. Atemmaske nach Anspruch 6, **dadurch gekennzeichnet, dass** der Maskenkörper (1), der Trägerarm (3), das Kissen (5) und die zwei Seitenarme (2a, 2b) integriert und aus einem einzigen Teil geformt sind, das aus einem elastischen weichen Material hergestellt ist.

8. Atemmaske nach Anspruch 6, **dadurch gekennzeichnet, dass** die zwei gegenüberliegenden, seitlich vom Maskenkörper (1) projizierenden Seitenarme (2a, 2b) jeweils Verbindungsmittel für das Kopfgestell (11) zur Befestigung eines Kopfgestells umfassen, wobei die Verbindungsmittel für das Kopfgestell (11) vorzugsweise mindestens ein durchgehendes Loch oder Schlitz umfassen.

9. Atemmaske nach einem der vorausgehenden Ansprüche, **dadurch gekennzeichnet, dass** der Maskenkörper (1) eine allgemein dreieckige dreidimensionale Form hat.

10. Atemmaske nach einem der vorausgehenden Ansprüche, **dadurch gekennzeichnet, dass** die Atemmaske eine Nasenmaske ist, vorzugsweise eine pädiatrische Nasenmaske, die so dimensioniert ist, dass sie auf die Nasenregion eines Säuglings oder Kindes passt.

11. Baugruppe, umfassend eine Gasabgabevorrichtung und eine Atemmaske nach einem der vorausgehenden Ansprüche, wobei die Gasabgabevorrichtung vorzugsweise mit der Atemmaske über eine Gasleitung, wie einen flexiblen Schlauch verbunden ist.

## Revendications

1. Masque respiratoire comprenant un corps de masque (1) et un bras de maintien (3) agencé sur le corps de masque (1) et faisant saillie vers le haut depuis ledit corps de masque (1), ledit bras de maintien (3) comprenant une extrémité libre (3c), le bras de maintien (3) comprenant en outre au moins une portion proximale (3b) et une portion distale (3a), ladite portion proximale (3b) étant fixée d'un seul tenant au corps de masque (1) et ladite portion distale (3a) comprenant l'extrémité libre (3c) dudit bras de maintien (3), la portion distale (3a) pouvant être détachée du bras de maintien (3), la portion proximale (3b) et la portion distale (3a) du bras de maintien (3) comprenant chacune des moyens de fixation (10, 15) pour fixer un harnais de tête, **caractérisé en ce que** :
- le corps de masque (1) et le bras de maintien (3) sont moulés en une seule pièce et composés d'un matériau mou élastique, et
- la zone intermédiaire (3d) entre la portion proximale (3b) et la portion distale (3a) comprend une portion col (14) formant un col ayant une première épaisseur (Tn) inférieure à une deuxième épaisseur (Tp) de la portion proximale (3b) et inférieure à une troisième épaisseur (Td) de la portion distale (3a).

2. Masque respiratoire selon la revendication précédente, **caractérisé en ce que** la portion proximale (3b) et la portion distale (3a) sont séparées par une zone intermédiaire (3d) comprenant un emplacement de détachement de la portion distale (3a).

3. Masque respiratoire selon l'une quelconque des revendications précédentes, **caractérisé en ce que** la portion col (14) comprend l'emplacement de détachement de la portion distale (3a).

4. Masque respiratoire selon l'une quelconque des revendications précédentes, **caractérisé en ce que** le matériau mou élastique comprend de la silicone.

5. Masque respiratoire selon l'une quelconque des revendications précédentes, **caractérisé en ce que** les moyens de fixation (10, 15) sont des trous ou fentes traversants.

6. Masque respiratoire selon l'une quelconque des revendications précédentes, **caractérisé en ce que** le corps de masque (1) comprend en outre :
- une chambre interne (6) et un orifice d'entrée (4) en communication fluidique avec la chambre interne (6),
- un coussinet flexible (5) ayant une ouverture (7) adaptée pour recevoir au moins une partie du nez du patient, quand le patient porte le masque, et
- deux bras latéraux (2a, 2b) agencés de chaque côté du corps de masque (1) et faisant saillie latéralement depuis ledit corps de masque (1).

7. Masque respiratoire selon la revendication 6, **caractérisé en ce que** le corps de masque (1), le bras de maintien (3), le coussinet (5) et les deux bras latéraux (2a, 2b) sont intégrés et formés d'une seule pièce composée d'un matériau mou élastique.

8. Masque respiratoire selon la revendication 6, **caractérisé en ce que** les deux bras latéraux opposés (2a, 2b) faisant saillie latéralement depuis ledit corps de masque (1) comprennent chacun un moyen de connexion au harnais de tête (11) pour fixer un harnais de tête, le moyen de connexion au harnais de tête (11) comprend de préférence au moins un trou ou une fente traversant.

9. Masque respiratoire selon l'une quelconque des revendications précédentes, **caractérisé en ce que** le corps de masque (1) a une forme tridimensionnelle triangulaire générale.

10. Masque respiratoire selon l'une quelconque des revendications précédentes, **caractérisé en ce que** le masque respiratoire est un masque nasal, de préférence un masque nasal pédiatrique dimensionné pour s'ajuster sur la région nasale d'un nourrisson ou d'un enfant.

11. Ensemble comprenant un dispositif d'alimentation en gaz et un masque respiratoire selon l'une quelconque des revendications précédentes, le dispositif d'alimentation en gaz est de préférence connecté au masque respiratoire à l'aide d'une conduite de gaz, tel qu'un tuyau flexible.
